# EUROPÄISCHE PATENTANMELDUNG

(11) **EP 2 404 581 A2**
(43) Veröffentlichungstag der Anmeldung: **11.01.2012**
(21) Anmeldenummer: 11172683.2
(22) Anmeldetag: 05.07.2011
(51) Int. Cl.: A61F 13/00

(54) **Absorptionskörper zur Auflage auf Wunden**

(30) Priorität: 09.07.2010 DE 202010008025 U
(71) Anmelder: McAirlaid's Vliesstoffe GmbH & Co. KG, 37339 Berlingerode (DE)
(72) Erfinder: Schmidt, Andreas, 37115 Duderstadt (DE)
(74) Vertreter: Christophersen, Ulrich Rudolf

(57) **Zusammenfassung**

Es wird ein Absorptionskörper zum Auflegen auf Wunden beansprucht, der aus einem mehrlagig Schichtkörper aufgebaut ist mit
A) einer der Haut zugewandten Lage A,
B) einer der Haut abgewandten Lage B und
C) einem zwischen der Lage A und der Lage B angeordneten Saugkörper und dadurch gekennzeichnet ist, dass die Lage A im Bereich der Wunde ein für Flüssigkeiten durchlässiges Material und die Lage B atmungsaktives Material aufweist. Der Absorptionskörper eignet sich insbesondere als Wundauflage.

## Beschreibung

Die Erfindung betrifft einen Absorptionskörper zum Auflegen von Wunden aus einem mehrlagig aufgebauten Schichtkörper mit einer der Haut zugewandten Lage A, einer der Haut abgewandten Lage B und einer zwischen diesen Lagen angeordneten Saugkörper.

Absorptionskörper zum Aufnehmen von Körperflüssigkeiten finden nicht nur in der Hygiene sondern auch in der Medizin Anwendung, insbesondere zur Behandlung von Wunden.

Absorptionskörper, die als Wundauflagen eingesetzt werden, werden in der Regel auf die äußere Wunde gelegt, um das Eindringen von Fremdkörpern in die Wunde zu verhindern und Blut und Wundsekret aufzunehmen.

Im Europäischen Patent 1 507 498 B1 wird ein Absorptionskörper zum Auflegen auf Wunden, insbesondere zum Aufsaugen von Wundflüssigkeiten, offenbart. Der dargestellte Absorptionskörper weist einen flachen Materialabschnitt aus Absorptionsmaterial, nämlich einem aufsaugenden Vlies mit darin verteilten Super-Absorberteilchen sowie eine flüssigkeitsdurchlässige Hülle auf. Diese flüssigkeitsdurchlässige Hülle umgibt den Materialabschnitt aus Absorptionsmaterial und bildet eine Barriere gegen feste Körperausscheidungen und verhindert den Durchtritt von anderen ausgetretenen Substanzen zu dem innerhalb der Hülle angeordneten Absorptionsmaterial. Der Materialabschnitt aus Absorptionsmaterial weist eine Fläche auf, welche kleiner ist als die Fläche der Umhüllung, nämlich in seinem nicht benetzten Zustand 3 % bis 75 % der flachgelegten Hülle. Die Fläche der flachgelegten Hülle ist ferner durch eine Naht und ggf. durch eine Faltkante begrenzt. Die Hülle und/oder das Absorptionsmaterial können an ihrem/seinem Umfang mit einer an dem Körper des Patienten haftenden Substanz, wie Klebstoff, versehen sein. Als geeigneter Klebstoff werden Pektin-Zellulose-Verbindungen genannt.

Die aus dem Stand der Technik bekannten Wundauflagen haben den Nachteil, dass sie auf der Wunde mit einem entsprechenden Verband fixiert werden müssen. Sie verfügen über keine geeigneten Mittel, mit Hilfe derer die Wundauflage direkt auf die Haut des Patienten fixiert werden kann.

Der vorliegenden Erfindung lag demgemäß die Aufgabe zugrunde, einen Absorptionskörper zum Auflegen auf Wunden zur Verfügung zu stellen, die eine volle Aufsaugkapazität aufweisen, jedoch auch ohne weitere Hilfsmittel, wie Verbandmaterial, auf der Wunde bzw. auf der Haut des Patienten fixiert werden können.

Gegenstand der vorliegenden Erfindung ist ein Absorptionskörper der eingangs beschriebenen Art, der sich dadurch auszeichnet, dass die Lage A im Bereich der Wunde ein für Flüssigkeiten durchlässiges Material und die Lage B atmungsaktives Material aufweist.

Der erfindungsgemäße Absorptionskörper eignet sich insbesondere zum Auflegen auf Wunden, d. h. auf sogenannte äußere Wunden, die im Allgemeinen auf der Haut einschließlich der Schleimhaut des Patienten auftreten können. Bei diesen Wunden kann es sich um akute Verletzungen oder auch um chronische Wunden, also nicht-heilende Wunden, bzw. nur durch sekundäre Wundheilung heilende Wunden handeln, aus denen z. B. Wundflüssigkeit austritt.

Ein weiterer Gegenstand der vorliegenden Erfindung ist demgemäß die Verwendung des Absorptionskörpers als Wundauflage.

Der erfindungsgemäße Absorptionskörper hat den Vorteil, dass die Wunde durch eine die austretende Körperflüssigkeit aufnehmende Lage bedeckt wird, die die austretende Flüssigkeit aufnimmt bzw. durch die die Flüssigkeit hindurchtritt und in den benachbarten Saugkörper transportiert, von welchem die Flüssigkeit absorbiert wird. Die der Haut abgewandten Lage weist den Vorteil auf, dass diese atmungsaktiv ist, d. h. für Wasserdampf durchlässig, für größere Moleküle oder Teilchen aber undurchlässig ist, Schweiß kann von innen nach außen entweichen. Die mit dem erfindungsgemäßen Absorptionskörper bedeckte Wunde ist nicht hermetisch verschlossen, es kann ein Luft- und Feuchtigkeitsaustausch erfolgen, Wundsekrete und andere Flüssigkeiten können jedoch nicht nach außen hindurchtreten und auch nicht von außen in Richtung Wunde gelangen. Dies hat auch den Vorteil, dass die atmungsaktive Lage eine Sperre auch für Bakterien etc., welche die Wunde infizieren könnten, darstellt.

Der erfindungsgemäße Absorptionskörper ist ein mehrlagig aufgebauter Schichtkörper, der üblicherweise im Wesentlichen aus einer der Haut zugewandten Lage A, einer der Haut abgewandten Lage B und einem Saugkörper, der zwischen diesen beiden Lagen angeordnet ist, besteht.

Die der Haut zugewandte Lage A liegt unmittelbar auf der Wunde und auf der die Wunde umgebenden Hautoberfläche auf. Um eine optimale Wundversorgung zu gewährleisten, ist die Lage A zumindest in dem Bereich, in welchem diese unmittelbar auf der Wunde aufliegt, aus flüssigkeitsdurchlässigem Material. Dieses flüssigkeitsdurchlässige Material kann ausgewählt sein aus Materialien wie Nonwoven oder Gewebe und/oder gelochter zwei- oder dreidimensionaler Folie, die vorzugsweise nicht mit Wunden verkleben. NonwovenMaterialien sind textil-ähnliche Materialien, die aus langen Fasern hergestellt sind und die durch chemische, mechanische, Hitze- oder Lösungsbehandlung miteinander verbunden sind. Es handelt sich hierbei um textile Flächengebilde aus einzelnen Fasern, die flüssigkeitsdurchlässig sind. Neben den Nonwoven (= Vliesstoffen) können auch gelochte oder strukturierte zwei- oder dreidimensionale Folien als Lage A eingesetzt werden. Die gelochten Folien weisen als zweidimensionale Ausgestaltung in der Fläche Löcher auf, die einen Flüssigkeitsdurchlass des Materials gewährleisten. Gelochte dreidimensionale Folien zeichnen sich dadurch aus, dass durch die Lochung das gelochte Material sich über die Ebene des Flächengebildes erstreckt, wodurch eine dreidimensionale Struktur entsteht. Durch die dreidimensionale Struktur verringert sich die Auflagefläche auf der Wunde, was die Wundheilung weiter positiv beeinflussen kann und ein Verkleben von getrockneten Exsudat verhindert. Die Materialien, aus welchen die Nonwovenmaterialien bzw. die gelochten Folien hergestellt sein können, sind vorzugsweise Polyolefin-Folien, wie Polyethylen- oder Polypropylen-Folien oder auch Naturstoffvliese.

In einer möglichen Ausgestaltung der vorliegenden Erfindung kann die Lage A im Bereich außerhalb der Wunde zumindest teilweise mit einem Klebstoff versehen sein. Dadurch kann der Absorptionskörper auf der Körperoberfläche fixiert werden.

Als Klebstoffe kommen alle denkbaren hautverträglichen Klebstoffe in Betracht wie sie bei der Herstellung von Pflastern üblich und bekannt sind. Vorzugsweise sollte der Klebstoff ein dauerhaft klebender Klebstoff sein, der auch nach einmaliger Verwendung, d. h. wenn er bereits auf die Hautoberfläche aufgebracht und wieder abgezogen wurde, wieder verwendbar ist. Auch sollte der Klebstoff vorzugsweise nach In-Kontakt-Kommen mit Wasser seine Klebkraft nicht verlieren. Ein bevorzugt eingesetzter Klebstoff ist sogenanntes untervernetztes Polyurethan, d. h. ein Polyurethan mit niedriger Kennzahl, eingesetzt werden. Untervernetzte Polyurethane werden durch Umsetzung von Polyisocyanaten mit langkettigen Polyolen, die vorzugsweise frei von kurzkettigen Anteilen sein sollen, erhalten.

Die Oberseite des erfindungsgemäßen Absorptionskörpers, d. h. die der Haut abgewandten Seite, wird von der Lage B gebildet. Zwischen den Lagen A und B ist der Saugkörper angeordnet. Die Lagen A und B sowie der Saugkörper können die gleichen Abmessungen aufweisen. In einer möglichen Ausgestaltung sind die Abmessungen des Saugkörpers geringer als die der Lagen A und B. In dieser Ausgestaltung wird der Saugkörper von den Lagen A und B quasi umschlossen. Vorzugsweise weisen die Lagen A und B weisen die gleichen Abmessungen auf und der Saugkörper ist kleiner als diese beiden Lagen A und B, sodass die Lagen A und B über den Saugkörper hinausragen. An diesen Überständen können die Lagen A und B miteinander verbunden sein. Um einzelnen Lagen miteinander verbinden zu können, können sie, je nach Material, aus welchem diese Lagen gebildet sind, miteinander verklebt, verschweißt, gesiegelt oder in sonstiger Weise verbunden sein. In einer möglichen Ausgestaltung sind die Maße einer der beiden Lagen A oder B größer als die der anderen Lage, der überstehende Rand kann so um den Rand der kleineren Lage umgeschlagen werden, wodurch die Ränder ebenfalls geschlossen werden können.

Eine optimale Wundversorgung ist insbesondere dann möglich, wenn die der Haut abgewandten Lage eine Lage aus atmungsaktiven Materialien ist. Die Materialien, aus welchen die Lagen A und B hergestellt sind, können gleiche oder voneinander verschiedene Materialien sein. Erfindungsgemäß ist die Lage B atmungsaktiv, d.h. sie ist für Wasserdampf (Schweiß) durchlässig nicht aber für Flüssigkeiten. Die Lage B kann beispielsweise eine atmungsaktive Folie sein, wie eine gelochte zwei- und/oder dreidimensionale Folie, eine atmungsaktives SMS, Nonwoven (Vliese) aus natürlichen oder synthetischen Fasern, und/oder ein Laminat aus unterschiedlichen Materialien, wie aus Nonwoven und atmungsaktiver Folie (BTBS-Filme =breathable film textile backsheet), wie sie üblicherweise für die Herstellung von Außenseiten von Inkontinenzprodukten bekannt sind.

Als dritte Lage weist der erfindungsgemäße Absorptionskörper einen Saugkern auf. Dieser ist zwischen den Lagen A und B angeordnet, vorzugsweise nach Art einer Hülle umgeben. Der Saugkern dient dazu, die aus der Wunde ausgetretenen Exsudate aufzunehmen und dauerhaft zu absorbieren. Eine Rücknässung in Richtung Wunde sollte vorzugsweise weitgehend vermieden werden. Der Saugkern wird daher vorzugsweise von einem Material gebildet, welches dazu in der Lage ist, Körperflüssigkeiten nicht nur zu absorbieren, sondern auch zu speichern, wie Zellstoff oder Zellstoff-verwandte Materialien sowie synthetische saugfähige Materialien. Der Saugkern ist vorzugsweise ein Vlies aus Zellstofffasern, wie ein Airlaid.

In einer bevorzugten Ausführungsform wird ein Saugkern eingesetzt, der nach dem im europäischen Patent 1 032 342 beschriebenen Verfahren hergestellt ist. Vorzugsweise wird ein Airlaid aus Zellstofffasern als Absorptionsmaterial eingesetzt. In einer bevorzugten Ausgestaltung der vorliegenden Erfindung weist der Saugkern über seine Fläche verteilte Prägebereiche, in denen die Fasern, vorzugsweise Zellstofffasern, stärker als in den übrigen Bereichen miteinander verpresst und hierdurch klebstoff- und/oder bindemittelfrei verbunden sind. In einer derartigen Ausführungsform ist der Saugkörper aus einer Faserstoffbahn aus Zellstoff-Fasern hergestellt, welche unter Erzeugung eines Prägemusters im Druckbereich bindemittelfrei, punkt- oder linienförmig kalandriert und verbunden sind.

Der Saugkern kann die Prägebereiche an der der Lage A und/oder der Lage B zugewandten Fläche aufweisen (mit den Prägebereichen versehen sein). Die Faserschicht des Saugkerns ist demnach so strukturiert, dass die Zellstofffasern außerhalb dieser diskreten Prägebereiche gelockert übereinander oder nur schwach aneinander haftend vorliegen, wohingegen sie in den Prägebereichen miteinander verpresst sind und eine innige Verbindung mit den jeweils benachbarten Zellstofffasern eingehen. Durch diese Ausgestaltung ist ein völliger Verzicht auf Klebstoffe und Bindemittel zur Bildung des Verbundes aus Zellstofffasern möglich, was ein einfaches und vollständiges Recycling ermöglicht. In den Prägebereichen haften die Fasern nicht lediglich aneinander. Vielmehr wird durch die Druckbeaufschlagung in den Prägebereichen erreicht, dass benachbarte Zellstofffasern in diesen Prägebereichen fest miteinander verbunden sind. Diese Verbindung vermag auch der Einwirkung durch Feuchtigkeit zu widerstehen, so dass sich der erfindungsgemäße Absorptionskörper durch mechanische Belastbarkeit auch in nassem Zustand auszeichnet.

Die Bereiche außerhalb der diskreten Prägebereiche, in denen die Fasern gelockert übereinander oder nur schwach einander haftend vorliegen, zeichnet sich durch eine gute Absorptionsfähigkeit aus. Die absorbierte Flüssigkeit wird von den Fasern aufgenommen und über die gesamte Fläche des Saugkerns verteilt und dort gehalten.

Um die Absorptionsfähigkeit des Saugkerns weiter zu steigern, kann dieser Teilchen aus superabsorbierenden Polymeren enthalten. Diese können beispielsweise direkt bei der Herstellung des Saugkerns mit eingearbeitet werden.

Der Saugkörper C ist zwischen den Lagen A und B angeordnet und vorzugsweise von den Lagen A und B nach Art einer Hülle umgeben. Der Saugkörper kann an einer oder an beiden der Lagen A und B fixiert oder lose von den Lagen A und B umgeben sein. Um ein Verrutschen des Saugkörpers innerhalb dieser Hülle möglichst zu vermeiden und sicherzustellen, dass der Saugkörper zumindest vollständig oder teilweise die Lage A an der Stelle bedeckt, an welcher diese auf der Wunde aufliegt, sollte der Saugkörper mindestens die gleiche Größe haben wie die Wunde. In einer bevorzugten Ausführungsform ist der Saugkörper etwas kleiner als die Lage A.

Die Lagen A und B sind vorzugsweise an ihren Außenkanten verschlossen. In einer möglichen Ausgestaltung der vorliegenden Erfindung sind die Lagen A und B gleich bzw. nahezu gleich und die Kanten sind miteinander verklebt, verschweißt oder versiegelt. Diese Verbindung der beiden Lagen kann unmittelbar an den Kanten der Lagen erfolgen oder aber in einem gewissen Abstand von der Kante, so dass sich quasi ein umlaufender Saum bildet. Auch eine Verklebung bis zum Saugkern hin ist denkbar.

In einer weiteren möglichen Ausgestaltung der vorliegenden Erfindung ist die Fläche einer der beiden Lagen A und B größer als die andere, die überstehenden Flächen der größeren Lage kann um die Kanten der kleineren Lage umgeschlagen werden, wobei ebenfalls eine den Saugkörper umschließende Hülle entsteht.

Um die Stabilität des erfindungsgemäßen Absorptionskörpers zu erhöhen, sind in einer bevorzugten Ausführungsform der vorliegenden Erfindung der Saugkörper der Lage C und die der Haut abgewandten Lage B adhäsiv miteinander verbunden, d. h. vorzugsweise verklebt. Das Verkleben der Lage B und C verhindert ein Verrutschen des Saugkörpers innerhalb der Wundauflage und auch die Festigkeit des Absorptionskörpers (Wundauflage) als solche. Es wird ein stabiler und belastbarer Produktkörper erhalten, der üblicherweise auch unter Belastung nicht platzt. Darüber hinaus bietet das Verkleben auch fertigungstechnische Vorteile, während ein umlaufender Kleberand nur geringe Fertigungstoleranzen erlaubt, sind diese bei teilweiser oder vollflächiger Verklebung erheblich höher.

Die vorliegende Erfindung wird anhand der beigefügten Figuren näher erläutert. Es zeigen:
- Fig. 1: eine Draufsicht auf den Absorptionskörper
- Fig. 2: eine Ausführungsform des Absorptionskörpers in der Schnittdarstellung
- Fign. 3 und 4: weitere Ausführungsformen.
- Figur 5: in vergrößerter Darstellung einen Schnitt durch den Saugkern,

- Figur 6: in perspektivischer Darstellung einen Abschnitt eines Saugkerns.

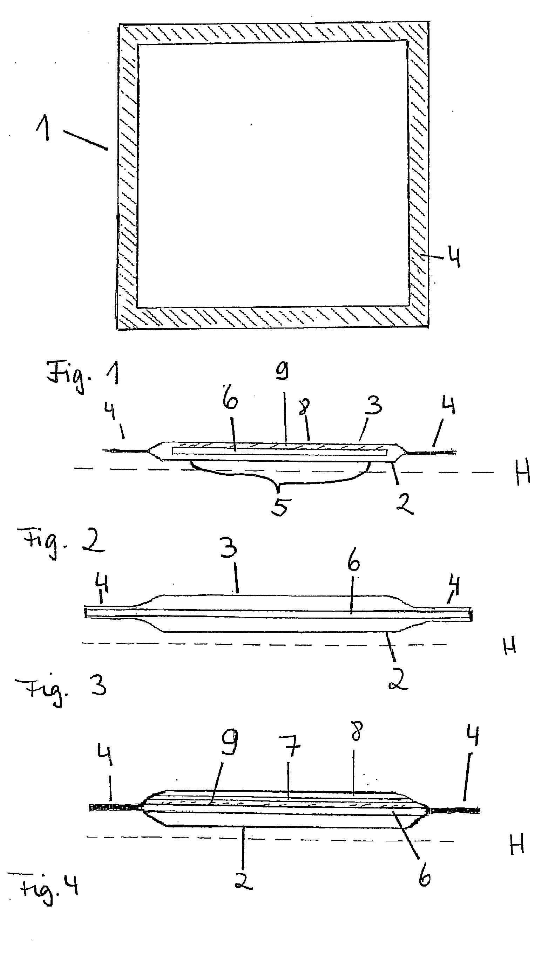

Fig. 1 zeigt den erfindungsgemäßen Absorptionskörper 1, wobei die der Haut zugewandte Lage 2 (Lage A) und ein umlaufender äußerer Bereich, in welchem die der Haut zugewandte Lage 2 und die der Haut abgewandte Lage 3 (Lage B) miteinander verbunden sind, zu sehen ist. Die der Haut zugewandte Lage 2 ist in ihren äußeren Bereichen 4 mit einem Klebstoff beaufschlagt. Der Klebstoff sollte möglichst nur in solchen Bereichen aufgebracht sein, die mit der Wunde selbst nicht in Berührung kommen. Um die Absorptionsfähigkeit des erfindungsgemäßen Absorptionskörpers nicht zu beeinträchtigen, sollten die Flächen der der Haut zugewandten Lage 2 möglichst frei von Beschichtungen etc. sein, so dass die Flüssigkeitsdurchlässigkeit dieser Lage erhalten bleibt.

In Fig. 2 ist eine mögliche Ausführungsform des erfindungsgemäßen Absorptionskörpers in einem schematischen Schnitt dargestellt. Der Absorptionskörper ist ein mehrlagig aufgebauter Schichtkörper 1 mit einer der Haut zugewandten Lage 2 und einer der Haut abgewandten Lage 3, die entlang ihrer Kantenbereiche 4 miteinander verbunden sind. Die Wundauflagefläche 5 ist flüssigkeitsdurchlässig, um Exsudate etc., die von der Wunde abgegeben werden, durchzulassen. Diese Exsudate werden vom Saugkörper 6 aufgenommen und dort gespeichert. Als weitere Lage enthält der Saugkörper auf der der Haut abgewandten Lage 3, die die Deckschicht des Absorptionskörpers darstellt. Die Deckschicht 3 wird von atmungsaktiven Materialien gebildet, d. h. aus solchen Materialien, die für Dampf durchlässig sind, aber flüssigkeitsundurchlässig. Durch diese der Haut abgewandte Deckschicht sollte möglichst vermieden werden, dass Exsudat aus den Absorptionskörper heraustritt und z. B. Kleidung verschmutzt. In der hier dargestellten Ausführungsform der Saugkörper 6 und die der Haut abgewandte Lage 3 miteinander verklebt, der Klebstoff ist hier als Klebstofflage 9 dargestellt.

In der in Fig. 2 dargestellten Ausführungsform sind die der Haut zugewandte Lage 2 und die der Haut abgewandte Lage 3 in dem Bereich 4 miteinander verbunden und bilden eine umlaufende Kante. Die Verbindung der Lagen kann punktförmig sein, so dass die Lagen 2 und 3 in den Außenbereichen 4 nicht vollständig miteinander verbunden sind. Sie können, wie es hier dargestellt ist, auch vollflächig miteinander verbunden sein. In dem Bereich 5 liegt der Absorptionskörper auf der Haut H auf, dieser Bereich erstreckt sich über die gesamte Wunde und vorzugsweise auch über den Bereich der Haut der unmittelbar zur Wunde benachbart ist und ggf. darüber hinaus. In der in Fig. 2 dargestellten Ausführungsform ist der Saugkörper 6 mit der Hautabgewandten Lage 3 verklebt.

In der in Fig. 3 dargestellten Ausführungsform erstreckt sich der Saugkörper bis in die Außenkanten 4, wo er beim Verbinden der Lagen 2 und 3 miterfasst wird und so zwischen diesen Lagen fixiert ist.

In Fig. 4 ist eine weitere Ausführungsform des erfindungsgemäßen Absorptionskörpers dargestellt. In dieser Ausführungsform ist die abgewandte Lage 3 aus einem Laminat aufgebaut. Dabei ist die atmungsaktive Lage 7 dem Saugkörper zugewandt, den Abschluss nach außen bildet ein Vlies 8. In einer möglichen Ausgestaltung kann die nach außen gerichtete Seite der atmungsaktiven Schicht 7 mit einem Muster, Logo oder ähnlichem bedruckt sein, welches im fertigen Laminat nach außen durchscheint. Die Lagen 7 und 8 können ebenfalls adhäsiv miteinander verbunden sein, beispielsweise miteinander verklebt sein. In der hier dargestellten Ausführungsform sind die der Haut abgewandte Lage 3 und der Saugkörper 6 miteinander verklebt, hier als Klebstofflage 9 dargestellt.

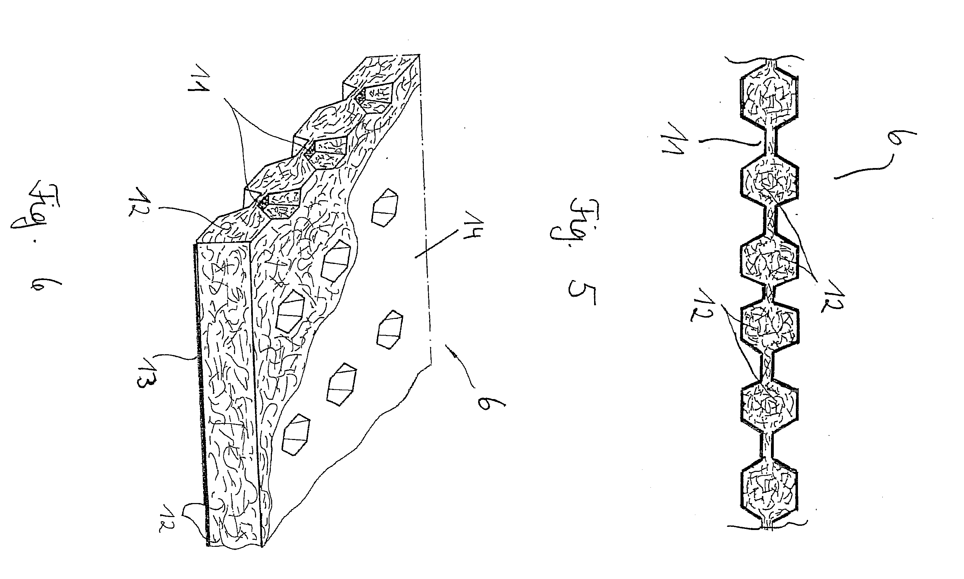

In den Figuren 5 und 6 sind in vergrößerter Darstellung eine mögliche Ausgestaltung des Saugkörpers 6 im Querschnitt und in Figur 6 eine perspektivische Darstellung eines Abschnitts eines Saugkörpers 6 dargestellt.

Die das Absorptionsverhalten für Flüssigkeiten, insbesondere Wundflüssigkeiten, bestimmende Schicht 10 besteht vorzugsweise aus Zellstofffasern 11. Die Zellstofffasern 11 sind in den Prägebereichen 12 verdichtet und auf diese Weise miteinander verbunden. In der hier dargestellten Ausführungsform liegen die Prägebereiche 12 sich auf der Ober- und Unterseite einander gegenüber, sodass im jeweiligen Prägebereich 12 ein nur noch schmaler Steg miteinander verbundener Zellstoffmasse verbleibt. Die übrigen, zwischen den Prägebereichen 12 angeordneten Bereiche der Schicht 10 weisen eine lockere Zellstoffschichtung auf. Eine innige Verbindung zwischen den Zellstofffasern 11 besteht in diesen Bereichen nicht. Die Prägebereiche 12haben in der hier dargestellten Ausführungsform die Gestalt von Pyramidenstümpfen oder Kegelstümpfen, wobei der Winkel der hierdurch gebildeten Schrägen zwischen 10° und 45° liegen sollte.

Als Zellulosematerial für die Schicht 10 lässt sich preiswert zur Verfügung stehendes Massenmaterial einsetzen. Vorzugsweise wird ein möglichst ungebleichter sogenannter "fluff pulp" eingesetzt, der sich, anders als gebleichte Zellstoffe, durch ein sehr gutes Bindungsverhalten auszeichnet, was die mechanische Festigkeit des Saugkörpers gegen vertikale Zugkräfte verbessert.

Bei der Herstellung in einem kontinuierlichen Verfahren wird die später die Schicht 10 bildende Faserstoffbahn aus einer im Luftstrom aufgeschichteten Schüttung von Zellstoffasern aus defiberisiertem Zellstoff (wood pulp) und ggf. eingestreuten Absorbermaterialien hergestellt. Die Faserstoffbahn aus Zellstofffasern wird durch schichtweises Schütten der Zellstofffasern erhalten. Werden weitere Absorbermaterialien, wie SAP, zugesetzt, so kann auch dieser Zusatz in einer schichtweisen Schüttung erfolgen, beispielsweise durch eine wechselnde Schüttung aus Zellstofffasern, Absorbermaterial, Zellstofffasern, so lange, bis die gewünschte Menge an Zellstoffasern und gegebenenfalls Absorbermaterial zugeführt wurde. In der in Figur 6 dargestellten Ausführungsform für einen Saugkörper weist die Lage 10 aus Zellstofffasern 11 an ihrer Ober- und/oder Unterseite jeweils eine weitere Lage 13, 14 auf. Diese Lagen 13, 14 sind vorzugsweise aus einem dünnen Tissue-Material. Dieses Material wird bereits bei der Herstellung des erfindungsgemäß verwendeten Saugkerns mit eingearbeitet, indem die Zellstoffschüttung auf das Tissue 13 aufgebracht wird und mit einem weiteren Tissue 14 abgedeckt wird. Die Schichtung aus Tissue und Zellstoff sowie bei einer gegebenenfalls weiteren Tissueschicht kann gemeinsam in einen Kalander eingeführt werden, mit dem ein Muster von punkt- oder linienförmigen Druckbereichen erzeugt wird. Dieses Verfahren ist z. B. im europäischen Patent 1 032 342 beschrieben.

Das Tissuematerial ist im Stand der Technik bekannt und kann im Handel als Bahnmaterial erhalten werden. Das Tissuematerial gibt dem Saugkörper 6 eine weitere Stabilität, es kann auch verhindern, dass feinteiliger Abrieb der Zellstoffasern und/oder der Absorbermaterialien durch die für Flüssigkeiten durchlässige Lage A in Richtung Wunde gelangt.

Für die Herstellung einer standardisierten, defiberisierten Ware kann auf die am Markt verfügbaren, nachwachsenden Holzrohstoffe zurückgegriffen werden.

Das Verfahren der Zellstoffschüttung als Ausgangsprodukt für die Schicht 10 ermöglicht eine trockene Verarbeitung der Zellstoffasern 11 und damit bei der anschließenden Prägung zwischen zwei Strukturwalzen eine sehr gute Fusionierung der Zellstoffasern in den diskreten Prägebereichen. Außerhalb der Prägebereiche 12 liegen die Fasern locker aneinander, was die Saugfähigkeit und die Flexibilität der Schicht 10 verbessert.

Das Saugverhalten der Schicht 10 ist im wesentlichen "trocken", d. h. solange eine vollständige Sättigung noch nicht erreicht ist, lässt sich die absorbierte Flüssigkeit nicht nach Art eines Schwammes wieder ausquetschen. Auch nach Gebrauch und solange das Absorptionsverhalten des Saugkörpers 6 nicht erschöpft ist, erscheint daher die erfindungsgemäße Absorptionskörper trocken.

Die Herstellung erfolgt aus Bahnmaterial, welches in einem kontinuierlichen Prozess gefertigt wird. In luftunterstützter Schichtung werden zunächst die Zellstoffasern und die SAP's zur Bildung der Schicht 10 gelegt. Anschließend erfolgt in einem Kalander mit zwei strukturierten Kalanderwalzen die Herstellung der Prägebereiche 12. In weiteren kontinuierlichen Schritten werden oben die Deckschicht 3 und unten die Basisschicht 1 angeordnet und mit der Kernschicht 10 verklebt. Das so hergestellte Bahnmaterial wird schließlich in einzelne, vorzugsweise rechteckige Felder geschnitten, die in einem weiteren Fertigungsschritt zur Herstellung des erfindungsgemäßen Absorptionskörpers zugeführt werden.

### Bezugszeichenliste

- 1: Absorptionskörper
- 2: der Haut zugewandte Lage A
- 3: der Haut abgewandte Lage B
- 4: Kantenbereich
- 5: Wundauflagefläche
- 6: Saugkörper
- 7: atmungsaktive Lage des Laminats
- 8: Vlies (Teil des Laminats)
- 9: Lage aus Klebstoff
- 10: Schicht aus Zellstofffasern
- 11: Zellstofffasern
- 12: Prägebereiche
- 13, 14: Tissue
- H: Haut

## Patentansprüche

1. Absorptionskörper zum Auflegen auf Wunden aus einem mehrlagig aufgebauten Schichtkörper mit
A) einer der Haut zugewandten Lage A,
B) einer der Haut abgewandten Lage B und
C) einem zwischen der Lage A und der Lage B angeordneten Saugkörper, **dadurch gekennzeichnet, dass** die Lage A im Bereich der Wunde ein für Flüssigkeiten durchlässiges Material und die Lage B atmungsaktives Material aufweist.

2. Absorptionskörper nach Anspruch 1, **dadurch gekennzeichnet, dass** die Lage A flüssigkeitsdurchlässig ist und ausgewählt ist aus Nonwoven, Gewebe und/oder gelochter zwei- oder dreidimensionaler Folie.

3. Absorptionskörper nach einem der Ansprüche 1 oder 2, **dadurch gekennzeichnet, dass** der Saugkern ausgewählt ist aus einem Vliesmaterial aus Zellstoff oder Zellstoffverwandten Materialien, die Flüssigkeit speichern.

4. Absorptionskörper nach Anspruch 3, **dadurch gekennzeichnet, dass** der Saugkern Teilchen aus superabsorbierenden Polymeren enthält.

5. Absorptionskörper nach einem der Ansprüche 1 bis 4, **dadurch gekennzeichnet, dass** das atmungsaktive Material der Lage B eine Folie ist, die ausgewählt ist aus gelochten Folien, atmungsaktiven SMS-Folien, Nonwoven aus natürlichen oder synthetischen Fasern, Laminaten aus Nonwoven und atmungsaktiven Folien und/oder BTBS-Folien.

6. Absorptionskörper nach einem der Ansprüche 1 bis 5, **dadurch gekennzeichnet, dass** die Lagen A und B den Saugkern C umhüllen und die Ränder der Lagen A und B geschlossen sind.

7. Absorptionskörper nach einem der Ansprüche 1 bis 6, **dadurch gekennzeichnet, dass** der Saugkern ein Flächengebilde ist mit Abmessungen, die kleiner sind als die Fläche der Lagen A bzw. B.

8. Absorptionskörper nach einem der Ansprüche 1 bis 7, **dadurch gekennzeichnet, dass** der Saugkern (6) aus Zellstoffasern mit über die Fläche des Saugkerns verteilten Prägebereichen (11) versehen ist, in denen die Zellstofffasern stärker als in den übrigen Bereichen miteinander verpresst und hierdurch klebstoff- und/oder bindemittelfrei verbunden sind.

9. Absorptionskörper nach Anspruch 8, **dadurch gekennzeichnet, dass** die Prägebereiche (11) kegelstumpf- oder pyramidenstumpfförmig sind.

10. Absorptionskörper nach einem der Ansprüche 8 oder 9, **dadurch gekennzeichnet, dass** der Saugkern (2) als absorbierende Schicht aus im Luftstrom aufgeschichtete Fasern aus defiberisiertem Zellstoff (wood pulp) enthält und dass oberhalb und unterhalb dieser Fasern eine Lage aus Tissue angeordnet ist.

11. Absorptionskörper nach einem der Ansprüche 1 bis 10, **dadurch gekennzeichnet, dass** auf der der Haut zugewandten Oberfläche der Lage A ein Material aus Nonwoven, Gewebe und/oder gelochter zwei- oder dreidimensionaler Folie (welche die Atmungsaktivität des darunterliegenden Materials nicht beeinträchtigt) angeordnet ist.

12. Absorptionskörper nach Anspruch 11, **dadurch gekennzeichnet, dass** das atmungsaktive Material der Lage E und das auf der Oberfläche der Haut abgewandten Lage weitere flächige Material miteinander verklebt sind.

13. Absorptionskörper nach einem der Ansprüche 1 bis 12, **dadurch gekennzeichnet, dass** die Lagen B und C miteinander verklebt sind.

14. Verwendung des Absorptionskörpers nach einem der Ansprüche 1 bis 13 als Wundauflage.
